(19) Europäisches Patentamt
European Patent Office
Office européen des brevets

(11) Publication number: **0 415 767 A2**

# (12) EUROPEAN PATENT APPLICATION

(21) Application number: **90309511.5**

(51) Int. Cl.[5]: **C07D 213/803**

(22) Date of filing: **30.08.90**

(30) Priority: **31.08.89 US 403277**

(43) Date of publication of application:
**06.03.91 Bulletin 91/10**

(84) Designated Contracting States:
**AT BE CH DE DK ES FR GB GR IT LI LU NL SE**

(71) Applicant: **HOECHST CELANESE CORPORATION**
**Route 202-206 North**
**Somerville, New Jersey(US)**

(72) Inventor: **Bodman, Michael P.**
**4422 Clear Fork Drive**
**Corpus Christi, Texas(US)**
Inventor: **Elango, Varadaraj**
**4175 Crenshaw Drive**
**Corpus Christi, Texas(US)**
Inventor: **Fritch, John R.**
**4334 Five Points Road**
**Corpus Christi, Texas(US)**
Inventor: **Larkin, Donald R.**
**442 Williamson**
**Corpus Christi, Texas(US)**
Inventor: **Mueller, Werner H.**
**13825 Eaglesnest Bay Drive**
**Corpus Christi, Texas(US)**

(74) Representative: **De Minvielle-Devaux, Ian Benedict Peter et al**
**CARPMAELS & RANSFORD 43, Bloomsbury Square**
**London WC1A 2RA(GB)**

## (54) Process for preparing pyridine carboxylic acid derivatives.

(57) A method for preparing pyridine carboxylic acid esters by reacting N-hydroxyamino derivatives of the formula:

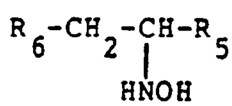

II

wherein $R_5$ and $R_6$ are each independently H, COZ, or CN, provided that they are not both H; or $R_5$ and $R_6$ together are $-CO-NR_{10}-CO$; Z is $OR_8$ or $NR_8R_9$; $R_8$ and $R_9$ are each independently H or alkyl or aryl; or $R_8$ and $R_9$ together with the nitrogen atom form an aliphatic or aromatic heterocyclic structures and $R_{10}$ is hydrogen, alkyl, aryl, hydroxy or an alkoxy of 1-6 carbon atoms; with an $\alpha,\beta$-unsaturated aldehyde or ketone is disclosed. In an additional embodiment, an alkene of formula $R_5-CH=CH-R_6$, wherein $R_5$ and $R_6$ are defined as above, is reacted with hydroxylamine or a salt thereof to form N-hydroxylamino derivatives which are then treated as above.

EP 0 415 767 A2

# PROCESS FOR PREPARING PYRIDINECARBOXYLIC ACID DERIVATIVES

## BACKGROUND OF THE INVENTION

Literature methods for preparing 5,6-dialkyl and 5-alkyl-6-arylpyridine-2,3-dicarboxylic acids and esters are limited and often require oxidation of alkyl or aryl substituents at positions 2 and 3 in order to obtain diacids. Recently there has been disclosed a method for the preparation of substituted and disubstituted pyridine-2,3-dicarboxylic acid esters and 2-alkylnicotinates utilizing $\alpha$-halo-$\beta$-ketoesters and $\alpha,\beta$-unsaturated aldehydes or ketones in the presence of an ammonium salt. The use of $\alpha$-halo-$\beta$-ketoesters is not desired due to the fact that such materials are usually costly and unstable.

U.S. Patent 4,723,011 discloses preparation of substituted and disubstituted pyridine2,3dicarboxylates by the reaction of an $\alpha$-halo-$\beta$-ketoester such as chloro-diethyloxalacetate (chloro-DOX) and an $\alpha,\beta$-unsaturated aldehyde or ketone such as 2-ethylacrolein in the presence of at least 2 molar equivalents of an ammonium salt in order to produce the desired compounds.

U.S. Patent 4,816,588 discloses and claims a process for preparing pyridine-2,3-dicarboxylic acids by the oxidation of 8-substituted quinolines.

European patent application 274,379 published July 13, 1988 discloses two processes for producing pyridine-2,3-dicarboxylic acid compounds. One process seems similar to that previously described in U.S. Patent 4,723,011 and the other process involves reacting on $\alpha,\beta$-unsaturated aldehyde or ketone with various aminomaleates or aminofumarates such as diethyl aminomaleate.

European patent application 299,362 published January 18, 1989 also discloses the same reaction.

Although the methods above-described are effective, nevertheless, because of the commercial importance of the compounds, particularly as useful intermediates for the preparation of herbicidal 2-(2-imidazolin-2-yl)nicotinic acids, esters and salts, any improvement in the process is of tremendous potential economic significance.

## SUMMARY OF THE INVENTION

It has now been found that N-hydroxyamino derivatives of Formula II can be reacted with an $\alpha,\beta$-unsaturated aldehyde or ketone to yield pyridinecarboxylic acid derivatives of Formula I

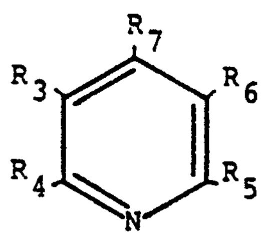

I

wherein $R_3$ is hydrogen, halogen, $C_1$-$C_6$ straight or branched alkyl, alkenyl, phenyl, or substituted-phenyl; $R_4$ and $R_7$ are each hydrogen, $C_1$-$C_6$ straight or branched alkyl, alkenyl, phenyl, or substituted-phenyl; $R_3$ and $R_4$ taken together can be $-(CH_2)-_{3-10}$; $R_5$ and $R_6$ are each independently H, $CO_2$, or CN, provided that they are not both or $R_5$ and $R_6$ together are $-CO-NR_{10}-CO$; Z is $OR_8$ or $NR_8R_9$; $R_8$ and $R_9$ are each independently H or alkyl (preferably $C_1$-$C_6$ straight or branched alkyl) or aryl; or $R_8$ and $R_9$ together with the nitrogen atom form aliphatic or aromatic heterocycic structures and $R_{10}$ is hydrogen, alkyl (preferably $C_1$-$C_6$ straight or branched alkyl), aryl, hydroxy or an alkoxy of 1-6 carbon atoms.

The expression substituted-phenyl is intended to mean phenyl substituted in one or more positions with halogen (bromine, chlorine, fluorine or iodine); alkoxy of 1-7 carbon atoms, cyano, nitro or amino.

The N-hydroxyamino derivatives useful in the novel process of this invention are those of the Formula II

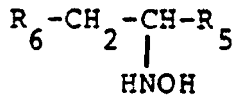

II

wherein $R_5$, and $R_6$ are described as in Formula I.

The $\alpha,\beta$-unsaturated aldehyde or ketone are those of Formula III.

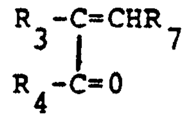

III

wherein $R_3$, $R_4$, and $R_7$ are as described in Formula I.

The acetal and ketal derivatives of Formula III or the Mannich base equivalent to formula III can also be used in the invention.

## CROSS REFERENCE TO RELATED APPLICATIONS

This application is related to application Serial No. 163,252, filed March 2, 1988, and Serial No. 214,549 filed July 1, 1988.

## DESCRIPTION OF THE REFERRED EMBODIMENTS

The reaction between the compounds of formula II and those of formula III is conveniently carried out by heating the same in the presence of an acid catalyst and suitable solvent preferably at reflux for periods of time ranging from 0.5 to 48 hours. Although the preferred temperature is at reflux, any temperature from ambient up to the boiling point of the solvent can be employed. A relative pH between 3-4 appears optimal although a pH ranging from 2-7 can be used.

The mole ratio of the compounds of formula II to the aldehydes or ketones of formula III is not critical and can range from about 1:3 to 3:1. It is preferred to use approximately 1:1.3 molar ratios.

If desired a dehydrogenation catalyst can be added to the reaction mixture in order to aid in aromatization of the newly-generated ring.

The dehydrogenation catalyst when employed is conventional in the art and includes metals or compounds of platinum, palladium ruthenium, iridium, nickel, iron, copper, antimony, cobalt, rhodium, etc. The dehydrogenation metal or compound thereof deposited on a suitable support, such as alumina, carbon, clay, zeolites, chromia, zirconia, etc. A preferred dehydrogenation catalyst is palladium on carbon.

As has been previously stated, an acid catalyst is employed. Suitable catalysts include inorganic acids such as hydrochloric, phosphoric, sulfuric, etc. and preferably organic acids such as acetic, trifluoroacetic,p-toluenesulfonic, methanesulfonic, trifluoromethanesulfonic, propionic, butyric or other carboxylic acids including aromatic carboxylic acids. Ion-exchange resins such as Amberlyst®, Dowex®, NAFION® can also be used as acidic catalysts.

When an acid is used which is also a solvent i.e. acetic acid, no additional solvent is required.

Solvents suitable for use in the method of this invention include: water, alcohols, chlorinated hydrocarbons, hydrocarbons, aromatic hydrocarbons, ethers, organic acids, esters, and aprotic solvents such as acetonitrile. The preferred solvents are lower alkyl alcohols, such as methanol, ethanol, propanol and butanol and aromatic hydrocarbons, such as benzene and toluene. A particularly preferred solvent is 1-butanol.

Thus, pyridinecarboxylic acid derivatives containing substituents in the 4-,5- and 6- position may conveniently be prepared by admixing Formula II N-hydroxyamino derivatives with a Formula III $\alpha,\beta$-unsaturated aldehyde or ketone in the presence of an acid and preferably a solvent, and stirring the resulting reaction mixture at a temperature in the range of ambient temperature to the boiling point of the solvent, and preferably at reflux, until the reaction is essentially complete and isolating the formed 4-substituted, 4-5-disubstituted, 4,6-disubstituted, 5-substituted, 6-substituted or 5,6-disubstituted pyridine-2,3-dicarboxylic acid derivatives by standard laboratory techniques such as extraction, evaporation, distillation or column chromatography.

Another embodiment o£ the invention involves the preparation of substituted and disubstituted pyridine-carboxylates of Formula I by reacting an alkene of Formula IV or IVA.

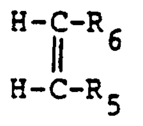

IV

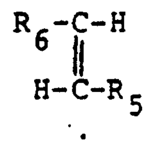

IVA

wherein $R_5$ and $R_6$ are defined above with an $\alpha,\beta$-unsaturated aldehyde or ketone of Formula III and hydroxylamine or a salt thereof such as the hydrochloride salt at elevated temperatures for periods of time ranging from 1 to 48 hours at a pH of 7-0 or even up to 12 and then lowering the pH to 2-7, or preferably 3-4.

A preferred embodiment of the invention involves the preparation of substituted and disubstituted pyridine-dicarboxylates of Formula I by treating a alkene of Formula IV or IVA wherein $R_5$ and $R_6$ are defined above with hydroxylamine or a mixture of a hydroxylamine salt and a base at a temperature of 15 to 60° C for periods of 0.1 to 2 hours at a pH of 7-9, then adding an $\alpha,\beta$-unsaturated aldehyde or ketone of Formula III and sufficient acid to take the pH to 2-7, preferably 3-4, and preferably with a solvent, and stirring the resulting mixture at a temperature in the range of ambient temperature to the boiling point of the solvent, until the reaction is essentially complete.

The reaction mixture is then cooled to ambient temperature of 20-40° C. The product is concentrated under reduced pressure and can be purified by conventional techniques such as distillation, extraction, evaporation, or column chromatography

If desired a dehydrogenation catalyst can be added to the reaction mixture.

The dehydrogenation catalyst when employed is conventional in the art and includes metals or compounds of platinum, palladium, ruthenium, iridium, nickel, iron, copper, antimony, cobalt, rhodium, etc. The dehydrogenation metal or compound thereof deposited on a suitable support, such as alumina, carbon, clay, zeolites, chromia, zirconia, etc. A preferred dehydrogenation catalyst is palladium on carbon.

When an acid is used which is also a solvent i.e. acetic acid, no additional solvent is required.

Solvents suitable for use in the method of this invention include: water, alcohols, chlorinated hydrocarbons, hydrocarbons, aromatic hydrocarbons, ethers, organic acids, esters, and aprotic solvents such as acetonitrile. The preferred solvents are lower alkyl alcohols, such as methanol, ethanol propanol and butanol and aromatic hydrocarbons, such as benzene and toluene. The particularly preferred solvent is 1-butanol.

In another embodient pyridine-2,3-dicarboxylic acid derivatives containing substituents in the 4-, 5- and 6-position may conveniently be prepared by reacting at a neutral or slightly basic pH Formula IV or IVA maleate or fumarate with hydroxylamine or a salt thereof, adding a formula III $\alpha,\beta$-unsaturated aldehyde, or ketone, at a pH of 2-7 with an acid and preferably a solvent, and stirring the resulting reaction mixture at a temperature in the range of ambient temperature to the boiling point of the solvent, and preferably at reflux, until the reaction is essentially complete and isolating the formed 4-substituted, 4,5-disubstituted, 4,6-disubstituted, 5-substituted, 6-substituted or 5-6-disubstituted pyridine-2,3-dicarboxylic acid derivatives by standard laboratory techniques such as extraction, evaporation column chromatography, or distillation.

The amount of hydroxylamine or salt thereof used ranges from about 1 to about 1.5 mols of hydroxylamine per mol of said maleate or fumarate. Preferred ranges are about 1-1.1 mols.

If a hydroxylamine salt is used, a base such as sodium hydroxide, potassium hydroxide or ammonium hydroxide, in an amount of 1 to 2 moles, preferably 1 to 1.2 moles per mole of said hydroxylamine salt is needed to liberate the hydroxylamine.

The mol ratio of the alkene of formula IV and IVA to the aldehyde or ketone of formula III is not narrowly critical and can range from about 1:3 to about 3:1. It is preferred to use approximately 1:1.3 molar ratios.

It is believed that the reaction of the IV and IVA maleates with the hydroxylamine or salt thereof inherently produces the N-hydroxyamino derivatives of Formula II.

## EXAMPLE 1

Preparation of Diethyl N-hydroxyaspartate (DENHA)

Hydroxylamine Free base (50% aq. soln., 45.0 g, 0.68 mol) was added dropwise to diethyl maleate (100.0 g, 0.56 mol) in a 3-neck flask blanketed with nitrogen. The reaction temperature was maintained below 55 °C with an ice bath. The mixture was stirred for 30 minutes. Dichloromethane (100 mL) was added to the reaction mixture and the organic layer was collected. The organic layer was concentrated under reduced pressure to give crude diethyl N-hydroxyaspartate (103 g, 89% yield). The product was analyzed by nuclear magnetic resonance spectroscopy (NMR) and shown to be at least 95% pure. NMR (acetone-d6) $\delta$ 1.20 (m, 6H), 2.59 (dd, J 6.8, 16.1 Hz, 1H), 2.76 (dd, J 6.8, 16.1 Hz, 1H), 3.89 (t, J 6.8 Hz, 1H), and 4.11 (m, 4H).

EXAMPLE 2

Preparation of DENHA from hydroxylamine sulfate

Aqueous sodium hydroxide (40%, 12.9 g, 0.129 mol) was added over 20 minutes to a mixture of diethyl maleate (17.3 g, 0.1 mol) and aqueous hydroxylamine sulfate (25%, 39.0 g, 0.059 mol). The reaction temperature rose from 28 °C to 53 °C during the addition. The reaction mixture was transferred to separatory funnel, methylene chloride (50 mL) was added to extract the aqueous product mixture, and the organic layer was separated and concentrated to give DENHA (18.5 g, 90% yield).

EXAMPLE 3

Preparation of Diethyl 5-Ethylpyridine-2,3-dicarboxylate (5-EPDC) from DENHA

DENHA (20.2 g, 0.1 mol) was dissolved in Benzene (100 mL) and stirred under nitrogen. Trifluoroacetic acid (2.0 g, 0.018 mol) and 2-ethylacrolein (9.8 g, 0.11 mol) were added and the reaction mixture was stirred at 72-75 °C for 16 hours. The reaction mixture was concentrated under reduced pressure to give crude diethyl 5-EPDC (27.92 g). The gas-liquid chromatographic (GLC) analysis of the crude product indicated that the reaction had proceeded with 91% conversion (based on diethyl maleate) and 41% yield (based on external standard) to diethyl 5-EPDC.

EXAMPLE 4

Preparation of Diethyl 5-EPDC from DENHA

DENHA (20.2 g, 0.1 mol) was dissolved in ethanol (38 mL) and stirred under nitrogen. Acetic acid (5.1 g, 0.085 mol) and 2-ethylacrolein (10.05 g, 0.12 mol) were added and the reaction mixture was stirred at reflux for 6 hours. The reaction mixture was concentrated under reduced pressure to give crude diethyl 5-EPDC (25.6 g). The GLC analysis of the crude product indicated that the reaction had proceeded with 94% conversion and 47% yield.

EXAMPLE 5

Procedure without Pd/C

Hydroxylamine free base (2.0 g, 0.031 mol) was added to a solution of diethylmaleate (4.3 g, 0.024 mol) in ethanol (15 mL) and the mixture was stirred for 30 min under nitrogen. The reaction products were analyzed by NMR and found to be 92% DENHA. Trifluoracetic acid (1.0 g, 0.009 mol) and hexadecane (0.5 g, 0.0002 mol) were added, and the reaction mixture was heated to 70° C. 2-Ethylacrolein (2.7 g, 0.032 mol) was added and the reaction mixture was refluxed for 5 hours. The reaction mixture was cooled to room temperature and analyzed by GLC. The analysis showed that the reaction had proceeded with 92% conversion (based on diethyl maleate) and 52% selectivity to diethyl 5-EPDC (based on hexadecane as an internal standard). The solvent was removed under reduced pressure to give the crude product (8.2 g, 48% yield).

## EXAMPLE 6

Synthesis of Diethyl 5-EPDC from Hydroxylamine Free Base 1 Diethyl Maleate, and 2-Ethylacrolein Procedure with Pd/C

Hydroxylamine free base (50% aq. soln., 8.0 g, 0.118 mol) was added dropwise to diethyl maleate (17.1 g, 0.1 mol) in a 3-necked 250-mL flask blanketed with nitrogen. The reaction temperature was maintained below 55° C with an ice bath. The mixture was stirred for 15 minutes, and then analyzed by NMR, which indicated 96% conversion of diethyl maleate to diethyl n-hydroxyaspartate (DENHA). Ethanol (40.0 g), 2-ethylacrolein (10.0 g, 0.12 mol), trifluroacetic acid (7.0 g, 0.06 mol), and 5% Pd/C (0.22 g, 0.1 mmol) were successively added to the reaction mixture, which was then refluxed for 6 hours under nitrogen. The reaction mixture was cooled to room temperature, filtered through a small column of celite to remove Pd/C and concentrated under reduced pressure to give the crude product (32 g). The crude product was purified by distillation to give diethyl 5-EPDC.

## EXAMPLE 7

Procedure with Acetic acid as Catalyst and Solvent

Hydroxylamine free base (50% aqueous solution, 68.03g, 0.12 mol) was added to diethyl maleate (17.8 g, 0.10 mol) at 25° C. The mixture was stirred for 15 min., then subjected to vacuum (0.25 mm Hg) for 15 min to remove water. Acetic acid (11.87 g, 0.18 mol) was added to bring the pH to about 3.8. 2-Ethylacrolein (10.05 g, 0.12 mol) was added and the reaction mixture was stirred for 5 hours at 105° C. The reaction mixture was cooled to room temperature and the crude product (46.5 g) analyzed by NMR. The analysis showed that the reaction proceeded with 95% conversion to give diethyl 5-EPDC in about 40% yield (based on external standard).

## EXAMPLE 8

Synthesis of Dibutyl 5-EPDC from Hydroxylamine, Dibutyl Maleate and 2-Ethylacrolein

Hydroxylamine free base (50% aq. soln., 8.0 g, 0.12 mol) was added dropwise to dibutyl maleate (25.0 g, 0.1 mol) in a 3-necked 250-ml flask blanketed with nitrogen. The reaction temperature was maintained below 55° C with an ice bath. The mixture was stirred for 30 min and then analyzed by NMR, which indicated 96% conversion of dibutyl maleate to dibutyl N-hydroxyaspartate. Butanol (29.8 g), hexadecane (0.98 g), 2-ethylacrolein (10.0 g, 0.12 mol), and trifluoroacetic acid (2.0 g, 0.018 mol) were added in succession to the reaction mixture, which was then stirred at 90-95° C for 4.5 hours under nitrogen. The

reaction mixture was cooled to room temperature and analyzed with hexadecane as an internal standard. The analysis showed that the reaction had proceeded with 82% conversion (based on dibutyl maleate) and 45% selectivity to dibutyl 5-EPDC. The solvent was removed under reduced pressure to give the crude product (37.8 g) which was purified by vacuum distillation to give dibutyl 5-EPDC (14.0 g, 35% yield).

## EXAMPLE 9

### Preparation of Diethyl 5-EPDC from Hydroxylamine Sulfate

Sodium hydroxide (40% aqueous solution, 13.0 g, 0.13 mol) was added over 15 minutes to a mixture of diethyl maleate (17.3 g, 0.100 mol) and hydroxylamine sulfate (25% aqueous solution, 39.10 g, 0.060 mol). The reaction temperature increased from 29°C to 45°C during the addition. After the reaction mixture had been stirred under nitrogen for an additional 30 minutes, 1-butanol (30.5 g) was added. The mixture was transferred to a separatory funnel, the layers were allowed to separate, and the organic layer (55.14 g) containing DEHNA was collected. The pH of the organic layer was found to be 7.3. Acetic acid (7.4 g, 0.123 mol) was added to the crude product from the above reaction to adjust the pH to 3.8. Ethylacrolein (9.84 g, 0.11 mol) was added dropwise to the reaction mixture over 15 min at room temperature. The reaction was slowly warmed to 95-96°C, stirred at this temperature for 20 hours, and then was concentrated under reduced pressure to give crude diethyl 5-EPDC (26.19 g). GLC analysis of this crude product indicated that the reaction had proceeded with 93% conversion to give diethyl 5-EPDC in about 51% yield.

## EXAMPLE 10

### Preparation of Diethyl 5-EPDC from Hydroxylamine Sulfate in Toluene

Sodium hydroxide (40% aqueous solution, 13.0 g, 0.13 mol) was added over 35 minutes to a mixture of diethyl maleate (17.3 g, 0.100 mol) and hydroxylamine sulfate (25% aqueous solution, 39.20 g, 0.060 mol). During the addition, the reaction temperature increased from 26°C to 55°C. After the reaction mixture had been stirred under nitrogen for 30 additional minutes toluene (36 mL) was added. The mixture was transferred to a separatory funnel, the layers were allowed to separate, and the organic layer (49.08) containing DEHNA was collected. The pH of the organic layer was found to be 6.6. Acetic acid (6.4 g, 0.106 mol) was added bo the crude product from the above reaction to lower the pH to 3.0. Ethylacrolein (9.88 g, 0.11 mol) was added dropwise to the reaction mixture over 15 min at room temperature. The pH of the resulting mixture was 3.3. The reaction mixture was warmed slowly to 79-80°C, stirred at this temperature for 20 hours, and then concentrated under reduced pressure to give crude diethyl 6-EPDC (25.67 g). GLC analysis of this crude product indicated that the reaction had proceeded with 94% conversion to give diethyl 5-EPDC in about 41% yield.

## EXAMPLE 11

### Procedure without added Acid catalyst

Hydroxylamine free base (50% aq. soln., 8.0 g, 0.12 mol) was added dropwise to diethyl maleate (17.4 g, 0.10 mol). The reaction mixture temperature was maintained below 55°C with an ice bath. The mixture was stirred for 30 minutes at room temperature (pH 7.35). Ethanol (35 g) and 2-ethylacrolein (9.8 g, 0.12 mol) were added to the reaction mixture. The pH of the reaction mixture was measured (6.75) and then the reaction mixture was refluxed for 20 hours under nitrogen. The mixture was cooled to room temperature and concentrated under reduced pressure to give 40.9 g of crude product. GLC analysis indicated 96% conversion of the diethyl maleate feed. Products included diethyl 2-aminomaleate (14.7%), diethyl

hexahydro-5-EPDC (1.1%), diethyl 5-EPDC (21.8%), and diethyl tetrahydro-5-EPDC6 (2.1%).

## EXAMPLES 12-40

The general procedure of Example 5 was repeated while varying solvent, catalyst, temperature, time, pH, and moles of reactants. Complete operating parameters and results are shown in the following table:

TABLE

PREPARATION OF Diethyl 5-EPDC

| Ex. No | Moles DEM | Moles NH2OH | Moles 2-EtAcr | Acid Name/g | Addit. Name/g | Solvent Name/g | Temp °C | Time hrs | Rx pH | Conv % | Anal. EPDC | Yield (%) 4H-EPDC |
|---|---|---|---|---|---|---|---|---|---|---|---|---|
| 12. | 0.099 | 0.133 | 0.134 | HOAc/15 | | EtOH/37.8 | 82 | 5.4 | 3.7 | 97.8 | 41.6 | 5.4 |
| 13. | 0.100 | 0.133 | 0.133 | HOAc/15 | | MeCN/30.0 | 65 | 5.3 | 3.5 | 98.0 | 36.1 | 11.9 |
| 14. | 0.025 | 0.030 | 0.024 | HOAc/2.1 | BQ/.2 | --- | 100 | 5.0 | 4.4 | 98.4 | 34.7 | 3.2 |
| 15. | 0.024 | 0.031 | 0.032 | TFA/1.0 | | EtOH/15.0 | 70-80 | 3.5 | 3.1 | 91.8 | 47.7 | 7.3 |
| 16. | 0.025 | 0.031 | 0.032 | TFA/1.0 | Pd-C/.3 | --- | 90-100 | 6.0 | 3.1 | 93.9 | 41.3 | 0.0 |
| Temperature | | | | | | | | | | | | |
| 17. | 0.100 | 0.121 | 0.120 | TFA/1.1 | | EtOH/49.0 | 50 | 5.0 | 4.0 | 88.8 | 15.5 | 1.0 |
| 18. | 0.101 | 0.139 | 0.135 | HOAc/ 15 | | EtOH/37.8 | 70 | 5.8 | 4.1 | 96.9 | 39.2 | 11.4 |
| 19. | 0.025 | 0.031 | 0.035 | TFA/1.5 | | EtOH/11.8 | 78 | 3.0 | 2.9 | 96.0 | 45.9 | 1.0 |
| 20. | 0.024 | 0.030 | 0.030 | TFA/2.8 | | --- | 90 | 5.0 | 3.0 | 96.7 | 39.5 | 0.0 |
| 21. | 0.100 | 0.121 | 0.119 | TFA/4.6 | | --- | 100 | 4.0 | 3.1 | 94.0 | 44.3 | 0.0 |
| Acids | | | | | | | | | | | | |
| 22. | 0.025 | 0.032 | 0.032 | TFA/1.2 | | --- | >100 | 4.0 | 3.1 | >99.0 | 36.9 | <1.0 |
| 23. | 0.101 | 0.102 | 0.119 | HOAc/5.1 | | --- | >100 | 5.5 | 3.8 | 98.8 | 38.9 | 1.0 |
| 24. | 0.054 | 0.030 | 0.032 | $H_2SO_4$/.45 | | EtOH/15.0 | 80 | 4.5 | 1.6 | 92.5 | 31.6 | 3.0 |
| 25. | 0.051 | 0.065 | 0.068 | $H_2SO_4$ | | EtOH/39.5 | 82 | 2.3 | 1.9 | >99.0 | 22.2 | 6.3 |
| 26. | 0.099 | 0.131 | 0.132 | p-TSA | | EtOH/30.0 | 82 | 4.3 | 3.0 | 66.4 | 9.6 | 1.5 |
| 27. | 0.101 | 0.131 | 0.133 | p-TSA | | EtOH/30.0 | 82 | 4.0 | 3.0 | 73.1 | 19.6 | 2.5 |
| 28. | 0.024 | 0.030 | 0.033 | PhOH/2.3 | | --- | 100 | 6.0 | 5.2 | 94.6 | 45.8 | 3.8 |
| 29. | 0.024 | 0.029 | 0.029 | DEAHC/2.6 | | EtOH/15.0 | 78 | 4.0 | 5.0 | 76.7 | 27.2 | 2.1 |
| Solvents | | | | | | | | | | | | |
| 30. | 0.024 | 0.030 | 0.027 | TFA/1.0 | | C6H6/10.0 | 65 | 4.0 | 3.4 | 95.8 | 44.9 | 0.0 |
| 31. | 0.024 | 0.025 | 0.027 | TFA/0.5 | | C6H6/20.0 | 80 | 20.0 | 4.0 | 93.6 | 41.4 | 0.0 |
| 32. | 0.100 | 0.131 | 0.133 | HOAc/15 | | EtOH/37.8 | 82 | 5.3 | 3.8 | 99.0 | 68.0 | 12.2 |
| 33. | 1.000 | 1.306 | 1.321 | HOAc/267 | | EtOH/300 | 82 | 5.0 | 3.5 | 90.8 | 42.3 | 6.3 |
| 34. | 1.014 | 1.320 | 1.324 | HOAc/137 | | EtOH/300 | 82 | 5.2 | 3.6 | 98.2 | 44.8 | 3.4 |
| 35. | 1.000 | 1.100 | 1.30 | HOAc/114 | | MeCN/300 | 83 | 5.3 | 3.6 | 95.4 | 41.3 | 9.1 |
| 36. | 0.100 | 0.131 | 0.133 | HOAc/15 | | Digly/38.0 | 105 | 3.0 | 3.8 | 98.2 | 37.4 | <0.7 |
| | | | | | | | | 19.0 | | | 40.8 | |
| Time | | | | | | | | | | | | |
| 37. | 0.100 | 0.133 | 0.133 | HOAc/20 | | EtOH/37.8 | 70 | 3.5 | 3.5 | 95.5 | 34.1 | 12.5 |
| | | | | | | | | 19.5 | | 98.9 | 44.6 | 8.9 |
| 38. | 0.099 | 0.134 | 0.131 | HOAc/15 | | EtOH/37.8 | 70 | 3.0 | 3.4 | 94.8 | 30.9 | 8.3 |
| | | | | | | | | 19.5 | | 98.6 | 40.5 | 7.3 |
| 39. | 0.100 | 0.110 | 0.130 | HOAc/15 | | MeCN/30.0 | 65 | 4.0 | 3.73 | 96.4 | 42.6 | 11.1 |
| | | | | | | | | 19.0 | | 96.9 | 49.3 | 10.4 |
| 10. | 0.100 | 0.131 | 0.133 | HOAc/15 | | EtOH/37.8 | 82 | 5.0 | 3.7 | 93.3 | 37.8 | 7.6 |

TABLE
(Continued)

Conversions and yields were determined by capillary gas chromatography using either an internal standard (n-hexadecane) or an external standard.

Notes:

In Examples 15 and 24, the reaction mixture was stirred a room temperature over 3 days.

Example 16 and 28, the reaction mixture was stirred at rc temperature overnight.

In Example 18, air was sparged through the reaction mixtu

In Examples 25-27, some insoluble salt was formed.

In Example 40, diethyl fumarate was used.

Acronyms:

| | | |
|---|---|---|
| DEM | = | Diethyl maleate |
| 2-EtAcr | = | 2-Ethylacrolein |
| Additi | = | Additive |
| Rx | = | Reaction |
| EPDC | = | Diethyl 5-Ethylpyridine-2,3-dicarboxylate |
| 4H-EPDC | = | Diethyl tetrahydro-5-ethylpyridine-2,3-carboxylate |
| TFA | = | Trifluoroacetic acid |
| BQ | = | Benzoquinone |
| p-TSA | = | p-toluenesulfonic acid |
| DEAHC | = | Diethylamine hydrochloride |

EXAMPLES 41-48

The procedure of Example 5 is repeated except that the following aspartates and aldehydes or ketones are used:

Aspartate

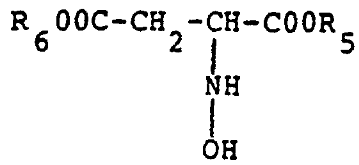

Aldehyde or Ketone

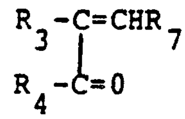

| | $R_6$ | $R_5$ | $R_3$ | $R_4$ | $R_7$ |
|---|---|---|---|---|---|
| Example 41 | methyl | propyl | H | H | phenyl |
| Example 42 | propyl | propyl | phenyl | ethyl | methyl |
| Example 43 | butyl | butyl | ethyl | methyl | H |
| Example 44 | ethyl | ethyl | methyl | H | H |
| Example 45 | ethyl | ethyl | H | methyl | H |
| Example 46 | ethyl | ethyl | H | H | methyl |
| Example 47 | ethyl | ethyl | —— $(CH_2)_3$ —— | | H |
| Example 48 | ethyl | ethyl | —— $(CH_2)_4$ —— | | H |

## Claims

1. A process for the preparation of pyridine-carboxylic acid derivatives of the formula I

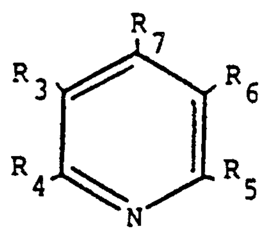

I

wherein $R_3$ is hydrogen, halogen, $C_1$-$C_6$ straight or branched alkyl, alkenyl, phenyl, or substituted-phenyl; $R_4$ and $R_7$ are each hydrogen, $C_1$-$C_6$ straight or branched alkyl, alkenyl, phenyl, or substituted phenyl; $R_3$ and $R_4$ taken together can be $-(CH_2)_{3-10}$; and $R_5$ and $R_6$ are each independently H, COZ, or CN, provided that they are not both H: or $R_5$ and $R_6$ together are $-CO-NR_{10}-CO$; Z is $OR_8$ or $NR_8R_9$; $R_8$ and $R_9$ are each independently H or alkyl or aryl; or $R_8$ and $R_9$ together with the nitrogen atom form an aliphatic or aromatic heterocyclic structure and $R_{10}$ is hydrogen, alkyl, aryl, hydroxy or an alkoxy of 1-6 carbon atoms; comprising reacting a N-hydroxyamino derivative of formula II:

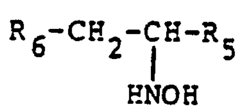

II

wherein $R_5$ and $R_6$ are as defined above with an $\alpha,\beta$-unsaturated aldehyde or ketone of formula III:

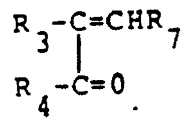

III

wherein $R_3$, $R_4$ and $R_7$ are as defined above in the presence of an acid and a solvent in a temperature range from ambient temperature to the boilding point of the solvent until the reaction is essentially complete.

2. A process according to claim 1 wherein the solvent is a $C_1$-$C_6$ alkyl alcohol.

3. A process according to claim 2 wherein the solvent is 1-butanol.

4. A process according to any of claims 1-3 wherein the reaction is carried out at a pH of 2-7.

5. A process according to claim 4 wherein the reaction is carried out at a pH of 3-4.

6. A process according to any of claims 1-5 wherein the N-hydroxyamino derivative is diethyl N-hydroxyaspartate.

7. A process according to claim 6 wherein the aldehyde is 2-ethylacrolein.

8. A process according to any of claims 1-5 wherein there is prepared diethyl 5-ethylpyridine-2,3-dicarboxylate, or diethyl 5-methylpyridine-2,3-dicarboxylate, or diethyl 6-ethylpyridine-2,3-dicarboxylate, or diethyl 4-methylpyridine-2,3-dicarboxylate, or diethyl 6-methylpyridine-2,3-dicarboxylate, or diethyl pyridine-2,3-dicarboxylate.

9. A process for the preparation of substituted and disubstituted pyridinedicarboxylates of formula I

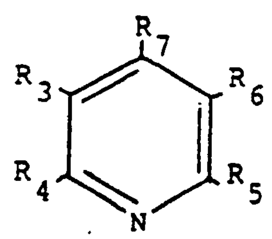

I

wherein $R_3$ is hydrogen, halogen, $C_1$-$C_6$ straight or branched alkyl, alkenyl, phenyl, or substituted-phenyl; $R_4$ and $R_7$ are each hydrogen, $C_1$-$C_6$ straight or branched alkyl, alkenyl, phenyl, or substituted phenyl; $R_3$ and $R_4$ taken together can be $-(CH_2)_{3-10}$; and $R_5$ and $R_6$ are each independently H, COZ, or CN, provided that they are not both H; or $R_5$ and $R_6$ together are $-CO-NR_{10}-CO$; Z is $OR_8$ or $NR_8R_9$; $R_8$ and $R_9$ are each independently H or alkyl or aryl; or $R_8$ and $R_9$ together with the nitrogen atom form an aliphatic or aromatic heterocycic structures and $R_{10}$ is hydrogen, alkyl, aryl, hydroxy or an alkoxy of 1-6 carbon atoms; comprising reacting an alkene derivative of formula IV and IVA:

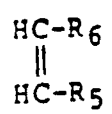

(IV)

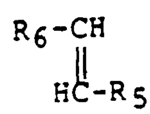

(IVA)

wherein $R_5$ and $R_6$ are as defined above with a hydroxylamine or salt thereof and then adding an $\alpha,\beta$-unsaturated aldehyde or ketone of formula III:

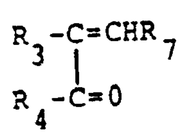

III

wherein $R_3$, $R_4$ and $R_7$ are as defined above and adding an acid and a solvent, heating to a temperature range of ambient temperature to the boiling point of the solvent until the reaction is essentially complete.

10. A process according to claim 9 wherein the solvent is a lower $C_1$-$C_6$ alkyl alcohol.

11. A process according to claim 10 wherein the solvent is 1-butanol.

12. A process according to any of claims 9-11 wherein the formula IV or IVA compound is diethyl maleate or diethyl fumarate.

13. A process according to any of claims 9-12 wherein the formula III compound is 2-ethylacrolein.

14. A process according to claim 13 wherein 2-ethylacrolein is added after said maleate or fumarate has reacted with said hydroxylamine or salt thereof and the pH is lowered to 3-4 with the addition of an acid.

15. A process according to any of claims 9-14 wherein the formula IV or IVA compound is reacted with hydroxylamine or a salt thereof at a pH of 7-9.

16. A process according to any of claims 9-15 wherein there is prepared diethyl 5-ethylpyridine-2,3-dicarboxylate, or dimethyl 5-ethylpyridine-2,3-dicarboxylate, or dibutyl 5-ethylpyridine-2,3-dicarboxylate.